# EUROPEAN PATENT APPLICATION

(11) **EP 1 634 960 A1**
(43) Date of publication of application: **15.03.2006**
(21) Application number: 04734148.2
(22) Date of filing: 20.05.2004
(51) Int. Cl.: C12Q 1/66, A01K 67/027

(54) **PROBES FOR ANALYZING INTERACTION BETWEEN PROTEINS AND METHOD OF ANALYZING INTERACTION BETWEEN PROTEINS USING THE SAME**

(30) Priority: 22.05.2003 JP 2003145466
(71) Applicant: Japan Science and Technology Agency, Kawaguchi-shi, Saitama 332-0012 (JP)
(72) Inventor: UMEZAWA, Yoshio, Tokyo 162-0063 (JP); KAIHARA, Asami, Tokyo 113-0023 (JP); OZAWA, Takeaki, Matsudo-shi, Chiba 270-2253 (JP); SATO, Moritoshi, Tokyo 113-0023 (JP)
(74) Representative: Owen, Deborah Jane
(86) International application number: PCT/JP2004/007245
(87) International publication number: WO 2004/104222

(57) **Abstract**

The present invention provides a pair of probes for analyzing protein-protein interactions, which comprises a probe A containing at least an N-terminal half polypeptide of split *Renilla* luciferase, and a probe B containing at least the remaining C-terminal half polypeptide of split *Renilla* luciferase.

## Description

### Technical Field

The present invention relates to a pair of probes for analyzing protein-protein interactions that detects where and when a protein-protein interaction occurs, and quantifies the amount of protein-protein interactions in living cells, and relates to a method for analyzing protein-protein interaction using the pair of probes. More specifically, the present invention relates to a pair of probes for analyzing protein-protein interactions that enables high-precision and high-speed screening of an intracellular signal reinforcing/suppressing substance participating in various protein-protein interactions in living cells or individuals, and a method for analyzing protein-protein interactions using the probe.

### Background Art

Protein-protein interactions are known to play key roles in structural and functional organization of living cells. It is also known that protein-protein interactions are engaged in a genetic transcription mechanism or intracellular signal transduction.

As a method for analyzing protein-protein interactions, split enzyme methods have been reported (Rossi, F., Charlton, C.A. and Blau, H.M., Proc. Natl. Acad. Sci. USA, 94, 8405-8410(1997); Remy, I., and Michnick, S.W., Proc. Natl. Acad. Sci. USA, 96, 5394-5399(1999); Pelletier, J.N., Arndt, K.M., Pluckthun, A., and Michnick, S.W., Nature Biotech. 17, 683-690(1999); Karimova, G., Pidoux, J., Ullmann, A., and Ladant, D., Proc. Natl. Acad. Sci. USA, 95, 5752-5756). According to these methods, a cleaved protein is complemented by a protein-protein interaction to restore its enzymatic activity, which is measured by expression type of bacteria or cells, or a fluorescent enzyme substrate; thereby the protein-protein interaction can be detected.

Such split enzyme methods are however accompanied by the drawbacks that not only the enzymatic reaction needs much time but also where or when the protein-protein interaction occurs in a cell cannot be identified because the stable-fluorescent product of the enzymatic reaction freely diffuses in the cell due to the stability thereof.

The present inventors have therefore made, as a probe capable of analyzing protein-protein interactions of any proteins with high precision in a convenient manner, a pair of probes for analyzing protein-protein interactions, in which firefly luciferase or GFP is produced and the enzymatic activity of firefly luciferase or fluorophore of GFP is regenerated by a protein splicing due to the protein-protein interaction (Japanese Patent Application No. 2000-224939; PCT/JP00/09348).

However, it is difficult to identify when and where the protein-protein interaction occurs even by using a pair of probes for analyzing protein-protein interactions, since the fluorescent product of enzymatic reaction or fluorophore of GFP is diffusive.

The purpose of the present invention is to overcome the problems of the conventional technologies and provide a probe for detecting where and when a protein-protein interaction occurs, and quantifying the amount of a protein-protein interaction in living cells; and a method for analyzing protein-protein interactions using the probe.

### Disclosure of the Invention

The first aspect of the present invention is a pair of probes for analyzing protein-protein interactions, which comprises:
a probe A containing at least an N-terminal half polypeptide of split *Renilla* luciferase; and
a probe B containing at least the remaining C-terminal half polypeptide of split *Renilla* luciferase.

The second aspect of the present invention is the one related to the above-described pair of probes, wherein the probe A contains an N-terminal half polypeptide of an intein and N-split *Renilla* luciferase, and the probe B contains a C-terminal half polypeptide of the intein and C-split *Renilla* luciferase.

The third aspect of the present invention is the one related to the above-described pair of probes, wherein a linker sequence is linked to each of the N-terminal half polypeptide of split *Renilla* luciferase and the remaining C-terminal half polypeptide of split *Renilla* luciferase. The fourth aspect of the present invention is the one related to the said pair of probes, wherein the linker sequence consists of 3 to 20 amino acid residues.

The fifth aspect of the present invention is the one related to any one of the above-described pair of probes, wherein the N-terminal half polypeptide of split *Renilla* luciferase and the remaining C-terminal half polypeptide of split *Renilla* luciferase are obtained by splitting *Renilla* luciferase between Ser91 and Tyr92.

The sixth aspect of the present invention is a method for analyzing protein-protein interactions, which comprises
fusing a protein "a" to any one of the above-described probe A, and fusing a protein "b" to any one of the above-described probe B;
making the protein "a" fused to the probe A and the protein "b" fused to the probe B coexist in the presence of coelenterazine and oxygen; and
measuring luminescence thus emitted.

The seventh aspect of the present invention is the one related to the above-described method, which comprises introducing a polynucleotide expressing the protein "a" fused to the probe A and a polynucleotide expressing the protein "b" fused to the probe B into cells, thereby making the protein "a" fused to the probe A and the protein "b" fused to the probe B coexist in the presence of coelenterazine and oxygen.

The eighth aspect of the present invention is the one related to the above-described method, which comprises introducing a polynucleotide expressing the protein "a" fused to the probe A and a polynucleotide expressing the protein "b" fused to the probe B into a non-human totipotent cell, causing ontogenesis of the cell to non-human animal, thereby making the protein "a" fused to the probe A and the protein "b" fused to the probe B coexist in the presence of coelenterazine and oxygen in any one of the cells of the animal or offspring animal thereof.

The ninth aspect of the present invention is a non-human animal or offspring animal thereof, which is obtained by
introducing a polynucleotide expressing the protein "a" fused to the probe A and a polynucleotide expressing the protein "b" fused to the probe B into a non-human totipotent cell; and
causing ontogenesis of the cell to non-human animal.

The tenth aspect of the present invention is a method for screening a substance, which comprises:
introducing a test sample into the above-described non-human animal or offspring animal thereof; and
analyzing a protein-protein interaction in the cell of the non-human animal or offspring animal thereof.

### Brief Description of Drawings

FIG. 1 is a schematic representation for one example of the pair of probes for analyzing protein-protein interactions according to the present invention and the working principle thereof.
FIG. 2 is a schematic representation for another example of the pair of probes for analyzing protein-protein interactions according to the present invention and the working principle thereof.
FIG. 3 is a schematic representation for the amino acid sequence and split site of *Renilla* luciferase used in the examples of the present invention.
FIG. 4 is a schematic representation of domain structures of split *Renilla* luciferase fusion protein (sRL) used in example of the present invention (a: hRL124C/A, b: sRL, c: N-terminal sRL91, d: C-terminal sRL91, e: sRL9lF).
FIG. 5 illustrates the relationship between the split site of *Renilla* luciferase and luminescence intensities generated by the protein-protein interaction in the example of the present invention (a: ratios of luminescence intensities with insulin to those without insulin, b: luminescence intensities of each pair of probes for analyzing the protein-protein interaction relative to luminescence intensities by full-length *Renilla* luciferase (hRL124C/A)).
FIG. 6 illustrates, in the example of the present invention, the luminescence intensities in the presence or absence of insulin when sRL91F is used (a: sRL91F, b: only N-terminal sRL91, c: only C-terminal sRL91F).
FIG. 7 illustrates, in the example of the present invention, with different concentration of insulin and the time course of the interaction between Y941 and SH2n when sRL91 is used.
FIG. 8 is, in the example of the present invention, immunoblot analysis of time-dependent tyrosine phospholylation on Y941 in sRL91.
FIG. 9 is microscopic images of CHO-IR cells in the example of the present invention (a: without insulin stimulation in CHO-IR cells expressing sRL91, b: 10⁻⁷ M insulin stimulation, c: CHO-IR cells expressing full-length *Renilla* luciferase (hRL124C/A)).
FIG. 10 is a schematic representation for an interaction mechanism between adapter proteins Shc and Grb.
FIG. 11 is results of the example of the present invention, which illustrates quantitative evaluation of the interaction between Grb2 and each Shc mutant of which tyrosine residues at three sites are replaced with phenylalanine residue and serine residue in the PTB domain is replaced with proline residue (a: EGF, b; E2, C: DHT, d: DES added).

### Reference numerals in these drawings denote:

1: A pair of probes for analyzing protein-protein interactions
1a: Probe A
1b: Probe B
1c: Probe C
1d: Probe D
2: *Renilla* luciferase
2a: N-split *Renilla* luciferase
2b: C-split *Renilla* luciferase
3a: Protein "a"
3b: Protein "b"
4: Bioluminescence
5a: Linker sequence
5b: Linker sequence
6a: N-terminal polypeptide of intein
6b: C-terminal polypeptide of intein
I: Coexistence
II: Interaction
III: Complementation
III': Protein splicing and excision
IV: Oxidative decomposition
V: Luminescence

### Best Mode for Carrying out the Invention

The pair of probes for analyzing protein-protein interactions of the present invention is characterized by the use of *Renilla* luciferase as an indicator substance.

*Renilla* luciferase (Lorenz, W.W., et al., Proc. Natl. Acad. Sci. USA, 88, 4438-4442(1991)) (SEQ ID NO: 1) is a monomeric protein having a molecular weight of 36 kDa, one half the molecular weight of that of firefly luciferase, and is known to easily express within mammalian cells. Although the crystal structure of *Renilla* luciferase is unknown, it is known that N-terminal and several cysteine residues are important for its luminescence activity (Paulmurugan, R., et al., Proc. Natl. Acad. Sci. USA, .99, 15608-13(2002), Liu, J., et. al., Gene 203, 141-148(1997)). Different from firefly luciferase, the enzymatic reaction of *Renilla* luciferase does not require ATP (Liu, J. & Escher, A., Gene 237, 153-159(1999)). Its substrate coelenterazine is known to penetrate through the mammalian cell membrane and rapidly diffuse throughout the cytozol (Contag, C.H. & Bachmann, M.H., Annu. Rev. Biomed. Eng., 4, 235-260(2002); Greer, L.F. & Szalay, A.A., Luminescence, 17, 43-74(2002)).

*Renilla* luciferase catalyzes the oxidation of coelenterazine in the presence of dissolved oxygen to its excited-state product (oxycolenterazine monoanion) to emit luminescence with a broad band (400 nm-630 nm) covering a tissue-transparent near infrared region, thereby yielding coelenteramide and carbon dioxide.

Taking advantage of the above characteristics of the *Renilla* luciferase, the present inventors have come up with the idea of realizing a probe that is capable of detecting when and where a protein-protein interaction occurs in living cells.

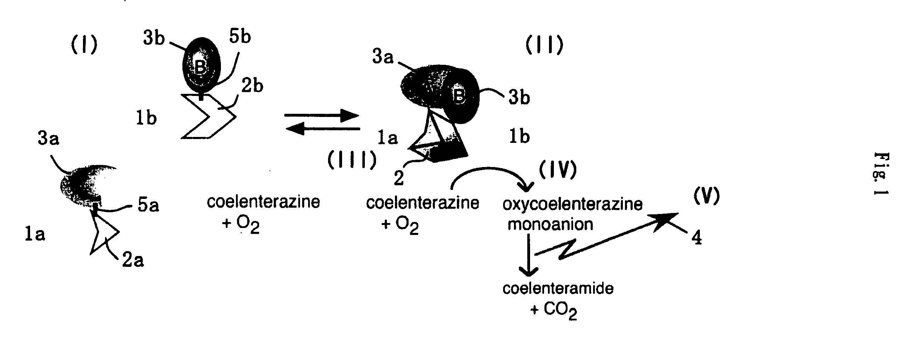

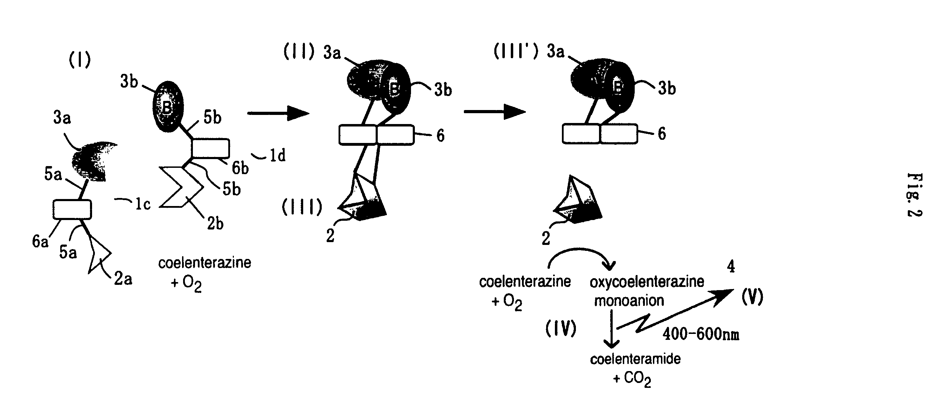

Constitution and working principle of the pair of probes for analyzing protein-protein interactions of the present invention are shown in FIGS. 1 and 2.

As illustrated in FIG. 1, the pair of probes ***1*** of the present invention comprises probe A ***1a*** containing at least an N-terminal half polypeptide of *Renilla* luciferase ***2a*** and probe B ***1b*** containing at least the remaining C-terminal half polypeptide of *Renilla* luciferase ***2b*** (hereinafter, the N- and C-terminal half polypeptides of *Renilla* luciferase may be denoted as "N-split *Renilla* luciferase" and "C-split *Renilla* luciferase", respectively).

To analyze the protein-protein interactions using the pair of probes of the present invention, an analysis-target protein "a" ***3a*** is fused to probe A ***1a*** and another analysis-target protein "b" ***3b*** is fused to probe B ***1b,*** and then they are co-existed in the presence of coelenterazine and dissolved oxygen (I). Interaction between protein "a" ***3a*** and protein "b" ***3b*** (II), and the consequent jaxtaposioning of N-split *Renilla* luciferase ***2a*** and C-split *Renilla* luciferase ***2b*** simultaneously lead to formation of the complemented *Renilla* luciferase ***2*** (III). In the presence of coelenterazine and oxygen in the system, the complement *Renilla* luciferase ***2*** catalyzed the oxidation of coelenterazine, which is a membrane permeable substrate, occurs (IV) and the energy of the carbonyl group of coelenterazine to the ground state is emitted as bioluminescence ***4*** spontaneously (V).

As described above, in the pair of probes ***1*** of the present invention, probe A ***1a*** and probe B ***1b*** may consist only of N- and C-split *Renilla* luciferases, respectively. And in addition, probe A *1a* and probe B *1b* may contain an N-terminal polypeptide of an intein **6a** and a C-terminal polypeptide of the intein ***6b***, respectively.

Described specifically, as illustrated in FIG. 2, in this case, in the pair of probes ***1,*** probe A contains N-terminal half polypeptide of the intein ***6a*** and N-split *Renilla* luciferase ***2a*** (which will hereinafter be called as probe A') ***1c,*** while probe B contains the C-terminal half polypeptide of the intein ***6b*** and the remaining C-split *Renilla* luciferase ***2b*** (which will hereinafter be called as probe B') ***1d.***

To analyze the protein-protein interactions using such a pair of probes, probe A' ***1c*** and probe B' ***1d*** are fused to the target proteins of analysis "a" ***3a*** and "b" ***3b,*** respectively (I). Interaction between the proteins ***3a*** and ***3b*** (II) induces the folding of N- and C-terminal halves of the intein and protein splicing reaction by the intein occurs. The split *Renilla* luciferases ***2a*** and ***2b*** are linked together by a peptide bond to reconstitute (splice) *Renilla* luciferase ***2*** (III').

In the presence of coelenterazine and oxygen in the system, the *Renilla* luciferase **2** catalyzed oxidation of coelenterazine, which is a membrane permeable substrate, occurs (IV) and the energy of the carbonyl group of coelenterazine to the ground state is emitted as bioluminescence **4** spontaneously (V). By detecting this bioluminescence **4**, the protein-protein interaction can be analyzed.

When the interaction (II) does not occur between the protein "a" ***3a*** and protein "b" ***3b***, the splicing reaction by the intein does not occur, *Renilla* luciferase ***2*** is not reconstituted (spliced) (III') and no marked change in bioluminescence ***4*** appears.

As the intein to be used in the pair of probes in the present invention, various known substances of biological origin can be used. Examples include substances derived from eukaryotic organisms typified by *Saccharomyces cerevisiae* (yeast) Sce VMA and Candida *tropiallis* (candida) Ctr VMA, those derived from eubacteria such as *Mycobacterium tuberculosis* (tubercle bacilli) Mtu recA, and those derived from archaebacteria such as *Thermoplasma asidophilum* Tac VMA.

In the pair of probes ***1c*** and ***1d*** of the present invention, the intein is preferably a site-specific endonuclease in order that autocatalytic excision of the intein occurs by the interaction between protein "a" ***3a*** and protein "b" ***3b.*** Moreover, in the pair of probes ***1c*** and ***1d*** in the present invention, two sites participating in the splicing in a protein precursor must be folded properly and juxtaposed precisely to each other in order that the splicing reaction by the intein occurs effectively (Duan, X., Gimble, F.S and Quiocho, F.A., Cell, 89, 555-564(1997)). An intein of biological origin may be used as it is, but it may be designed to facilitate splicing by substituting or deleting some amino acid residues or introducing a proper linker sequence.

The pair of probes in the present invention, probe A ***1a*** and probe B ***1b*** may consist only of N- and C-split *Renilla* luciferases, respectively, and in addition, probe A ***1a*** and probe B ***1b*** may have linker sequences ***5a*** and ***5b*** at their terminals, respectively. In the case of having linker sequences ***5a*** and ***5b,*** probe A ***1a*** and probe B ***1b*** are fused to proteins ***3a*** and ***3b*** via these linker sequences ***5a*** and ***5b,*** respectively. Although various sequences are given as examples of the linker sequences ***5a*** and ***5b*** and no particular limitation is imposed on them. Examples include those consisted of 3 to 20 amino acids sequences, more specifically, those having a glycine-alanine repeated sequence (FIG. 1).

Similarly, probe A' ***1c*** may consist only of the N-terminal half polypeptides of an intein ***6a*** and N-split *Renilla* luciferases ***2a,*** and it may further contain a linker sequence ***5a*** similar to those described above. Probe B' ***1d*** may also consist only of the C-terminal half polypeptides of an intein ***6b*** and C-split *Renilla* luciferases ***2b,*** and it may further contain a linker sequence ***5a.*** In other words, the N-terminal half polypeptides of intein ***6a*** and N-split *Renilla* luciferases ***2a*** may be linked directly or may be linked via linker sequences ***5a*** in probe A' ***1c,*** and the C-terminal half polypeptides of intein ***6b*** and C-split *Renilla* luciferases ***2b*** may be linked directly or may be linked via linker sequences ***5b*** in probe B' ***1d*** (FIG.2).

The N- and C-split *Renilla* luciferases ***2a*** and ***2b*** to be used in the pair of probes ***1*** of the present invention are the N-terminal half polypeptide and the remaining C-terminal half polypeptide obtained by splitting *Renilla* luciferase at a proper site. These N- and C-split *Renilla* luciferases ***2a*** and ***2b*** are complemented (III) by the interaction between protein "a" ***3a*** and protein "b" ***3b*** (II) (FIG. 1). And these N- and C-split *Renilla* luciferases ***2a*** and ***2b*** are directly linked and re-constitued with a peptide bond (III) due to the splicing reaction by intein ***6*** following by the interaction between the proteins "a" ***3a*** and "b" ***3b*** (FIG. 2).

In case of protein complementation, it is required that N- and C-split *Renilla* luciferases ***2a*** and ***2b*** do not exhibit luminescence activity before the complementation (III) (they exist individually) and restore enzymatic activity by the complementation. In the case of protein splicing, it is required that N- and C-split *Renilla* luciferases ***2a*** and ***2b*** do not exhibit luminescence activity before the reconstitution (splicing) (III')(they exist individually) and restore enzymatic activity by the re-constitution. For this purpose, the *Renilla* luciferase must be split at the position of cysteine (Cys), serine (Ser), or tyrosine (Tyr) so that the active centers are divided into two.

As described later in examples, according to the studies of the inventors, high-accuracy detection was possible by using N-split *Renilla* luciferase of probe A (or A') and C-split *Renilla* luciferase of probe B (or B') available by splitting the luciferase between Ser91 and Tyr92.

In the present invention, a protein-protein interaction is analyzed by using the pair of probes in the following manner: by fusing one probe (for example, probe A) to protein "a" which is one of the protein in the pair for analysis and fusing the other probe (probe B) to the other protein "b" which is the other protein in the pair, causing them to co-exist and detecting and measuring bioluminescence ***4***. Any method can be adopted in order to fuse each protein ***3a*** and ***3b*** to probe ***1a, 1b, 1c*** and ***1d*** insofar as it does not have any influence on the pair of target proteins or the pair of probes. For example, ordinarily employed chemical, biochemical or genetic engineering method can be employed.

In the present invention, an example of methods for making the pair of probes exist in the presence of coelenterazine and oxygen is that the pair of probes (***1a*** and ***1b,*** or ***1c*** and ***1d***) fused to proteins "a" ***3a*** and "b" ***3b*** is added to a solution containing coelenterazine. Using such a method, the protein-protein interaction can be detected and determined *in vitro.*

In the present invention, it is also possible to make the pair of probes exist in the presence of coelenterazine and oxygen by transfecting the expression vectors in cells. The expression vectors encode protein "a" which is fused to probe A ***1a*** and protein "b" which is fused to probe B ***1b***, or encode protein "a" ***3a*** which is fused to probe A' ***1c*** and protein "b" ***3b*** which is fused to probe B' ***1d.*** In this case, addition of oxygen is not necessary, because oxygen is there enough in the cells.

As the expression vector, a plasmid vector for the expression of animal cells is preferably employed. Such a plasmid vector can be introduced into a cell with a known methods, for example, electroporation, phosphorylated calcium method, liposome method or DEAE dextran method. By introducing the expression vectors for expressing proteins of analysis (***3a*** and ***3b***) which is fused to probes (***1a*** and ***1b,*** or ***1c*** and ***1d***) into the cells, it is possible to make the pair of probes (***1a*** and ***1b,*** or ***1c*** and ***1d**),* proteins (***3a*** and ***3b***), coelenterazine and oxygen exist together in the cell. Using such a method, the protein-protein interaction can be detected and determined *in vivo* without damaging the cell.

Moreover, according to the method for analyzing protein-protein interaction of the present invention, a polynucleotide expressing protein "a" ***3a*** fused to probe A ***1a*** and protein "b" ***3b*** fused to probe B ***1b,*** or a polynucleotide expressing protein "a" ***3a*** fused to probe A' ***1c*** and protein "b" ***3b*** fused to probe B' ***1d*** may be introduced into a non-human totipotent cell, and be caused ontogenesis of the cell to a non-human animal. By this method, the pair of probes ***1,*** proteins ***3,*** coelenterazine and oxygen are made coexist in all cells of the animal or offspring animal thereof. In this case, addition of oxygen is not necessary, because oxygen exists in the living body at a sufficient concentration.

The present invention also provides the thus available transgenic non-human animal. The transgenic non-human animal can be prepared in accordance with a known preparation method (for example, Proc. Natl. Acad. Sci. USA, 77, 7380-7384(1980)). Such a transgenic non-human animal has, in all the somatic cells thereof, the pair of probes ***1*** and proteins ***3*** so that an intracellular signal reinforcing/suppressive substance relating to the protein-protein interaction can be screened with high accuracy by introducing a sample substance such as pharmaceutical or toxic substance in the body of the animal and analyzing the protein-protein interaction in its cells and tissues.

In the method for analyzing protein-protein interaction according to the present invention, the protein-protein interaction can be confirmed by detecting luminescence (bioluminescence) ***4*** emitted by the above-described operation based on its working principle. The bioluminescence from *Renilla* luciferase has tissue permeability, so not only the timing but also the site of the protein-protein interaction can be identified by the observation and imaging of the luminescence in the cell. The protein-protein interaction can also be quantitatively determined by measuring a change of the luminescence intensity while varying conditions such as concentration or adding a specific sample. Moreover, it is possible to trace the protein-protein interaction from start to finish by measuring a time course of the luminescence intensity.

Embodiments of the invention will next be described in further detail in the following examples with reference to the accompanying drawings. It is needless to say that the invention is not limited to or by these examples, and details can be changed or modified.

### Examples

A known protein-protein interaction (Ozawa, T. et al., Anal. Chem. 73, 2516-2521(2001); Sato, M., et al., Nat. Biotechnol., 20, 287-294(2002); Sato, M., et al., Anal. Chem., 71, 3948-3954(1999)) between Y941 peptide (SEQ ID NO: 2) and N-terminal SH2 domain (SH2n) upon protein phosphorylation was confirmed using the pair of probes of the present invention.

In the following examples, reagents employed were as follows:

Restriction enzymes, modification enzymes and ligases were purchased from Takara Biomedicals (Tokyo, Japan). A synthetic renilla luciferase gene vector (hRL-CMV) coding renilla luciferase with most frequently used codons in mammals and renilla luciferase assay kits were purchased from Promega Co (Madison, WI). Mammalian expression vectors, pcDNA3.1(+) and pIRES, were obtained from Invitrogen (Groningen, Netherlands) and Clonetech (Palo Alto, CA), respectively. Ham's F-12 media, fetal bovine serum (FBS) and LipofectAMINE were obtained from Gibco BRL (Rockville, MD). Anti-myc antibody, anti-flag antibody and anti-phosphotyrosine antibody (PY20) were purchased from Santa Cruz Biotechnology, Inc. (Santa Cruz, CA). Alkaline-phosphatase labeled anti-rabbit and anti-mouse antibodies were purchased from Jacson ImmunoResearch Lab., Inc. (Pennsylvania, PA). A nitrocellulose membrane was obtained from Amersham Pharmacia Biotech (Buckinghamshire, UK). Chemiluminescent substrates of CDP-STAR were purchased from New England Biolabs, Inc. (Beverly, MA).

### Example 1

### Optimization of the pair of probes for analyzing protein-protein interaction

The 941 tyrosine residue in Y941 peptide derived from insulin receptor substrate-1 (IRS1) is phosphorylated by the insulin receptor upon insulin stimulation and dephosphorylated by a protein tyrosine phosphatase (Sato, M., et al., Anal. Chem. 71, 3948-3954(1999); Pratipanawatr, W., et al., Diabetes, 50, 2572-2578(2001)). The SH2n from p85 subunit (p85 330-429) of phosphatidylinositol 3-kinase binds with the phosphorylated 941-tyrosine residue within the IRS1 (Yoshimura, R., et al., Diabetes, 46, 929-936(1997); Golstein, B.J., et al., J. Biol. Chem. 1275, 4283-4289(2000)).

### (1) Plasmid construction

The gene of *Renilla* luciferase was split at eight sites near cysteine, serine and tyrosine to obtain two inactive fragments (N-split *Renilla* luciferase and C-split *Renilla* luciferase).

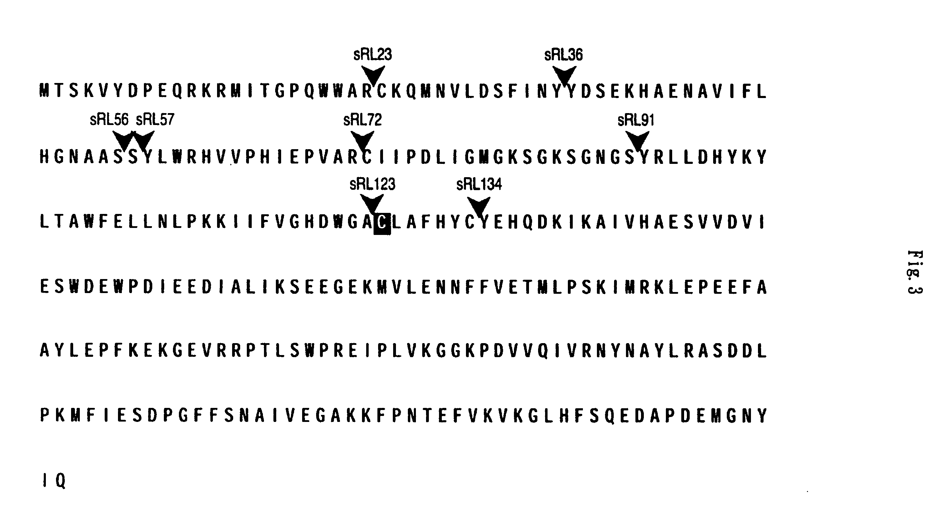

SEQ ID NO: 1 and FIG. 3 show the amino acid sequence of *Renilla* luciferase and split sites. In this Example, to increase luminescence intensity in accordance with the previous report (Liu, J., et al., Gene 203, 141-148(1997)), the 124-cysteine residue was replaced with an alanine residue in a *Renilla* luciferase mutant (hRL124C/A: SEQ ID NO: 3).

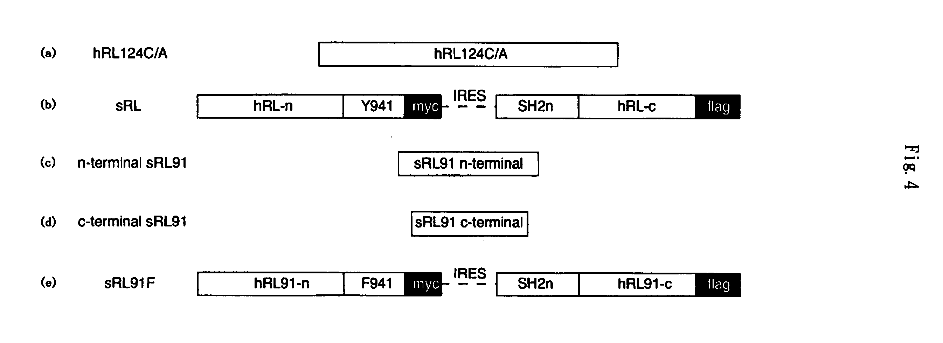

The N-split *Renilla* luciferase (hRLn) and C-split *Renilla* luciferase (hRLc) were fused to the proteins (Y941 and SH2n), respectively. The plasmid construct of each split *Renilla* luciferase-fused protein (sRL) is shown in FIG. 4. Each plasmid has a CMV-promoter sequence upstream of the initiation codon.

The amino acid sequence of Y941 is TEEAYMKMDLGPG (SEQ ID NO: 2) that is a tyrosine phosphorylated domain in IRS1. The SH2n is an N-terminal SH2 domain from p85 subunit of bovine phosphatidylinositol. In FIG. 4, indicated by a dotted line is an internal ribosomal entry site (IRES). Each sRL has a cassette consisting of [translation termination codon] - [internal ribosomal entry site] - [translation initiation codon].

To ensure that N- and C-split *Renilla* luciferases are spatially proximal when a protein-protein interaction occurs, a linker sequence (SEQ ID NO: 4) made of flexible 18 amino acid residues containing G-A repeat was inserted between the N-split *Renilla* luciferase and Y941 and another linker sequence (SEQ ID NO: 5) was inserted between the SH2n and C-split *Renilla* luciferase. Moreover, Myc and FLAG epitopes were inserted into the downstream of the N- and C-split *Renilla* luciferases.

*Escherichia coli* strain DH5a was used as a bacterial host for all plasmid construction subcloning. All plasmids were verified by sequencing with a genetic analyzer AB1 prism310 (PE Biosystems, Tokyo, Japan).

### (2) Cell culture and transfection

CHO-HIR cells were grown in Ham's F-12 supplemented with 10 % heat-inactivated FBS (Filtron), 100 unitlinl penicillin and 100 pg/mL streptomycin. Transfection was performed using LipofectAMINE 2000. The cells seeded in 6-well culture plates were transfected with 2 ng of the reporter plasmid. 6 h after the transfection, the medium containing LipofectAMINE was replaced with the Ham's F-12 supplemented with FBS, penicillin and streptomycin and incubated for 40 h to express the proteins.

### (3) Immunoprecipitation and immunoblotting analysis

CHO-HIR cells were stimulated with 100 nM insulin for 5 min at 37°C and were lysed mechanically with an ice-cold 2 x immunoprecipitation buffer (10 mM Tris-HCl (pH 7.4), 1 mM EDTA, 1 mM EGTA, 10 mM NaF, 0.2 mM sodium ortho-vanadate, 10 yg/mL leupeptin, 10 pg/ml pepstatin, 0.2 mlM phenylmethylsulfonyl fluoride (PMSF), 2 % IGEPALCA630, I % Triton X-100). The n-terminal fusion protein was immunogrecipitated from the whole cell lysates of the CHO-HIR cells with anti-myc antibody for 1 h at 4°C. Protein G-Sepharose 4 FF beads were used to absorb the immunoprecipitates and then washed fives times with an ice-cold immunoprecipitation buffer. The samples were separated by SDS-polyacrylamide gel electrophoresis and analyzed with the following antibodies: for Y941 phosphorylation, anti-phosphotyrosine antibody (1:500); for n-terminal protein assessment, anti-myc antibody (1:500); for c- terminal protein assessment, anti-flag anti body (1:1000).

### (4) Luminescence assay

CHO-HIR cells were stimulated with insulin at 37°C. Luminescence intensity was measured with a *Renilla* luciferase assay kit. Then the cells were washed twice with a cold PBS and lysed. The lysate was subjected to centrifugation at 15,000 g for 30 s at 4°C. A portion of the supernatant was measured for luminescence intensity with a Minilumat LB9506 luminometer (Berthold GmbH & Co. KG, Wildbad, Germany) for 10 s. The protein concentration in the supernatant was assessed by the Bradford method.

### (5) Results

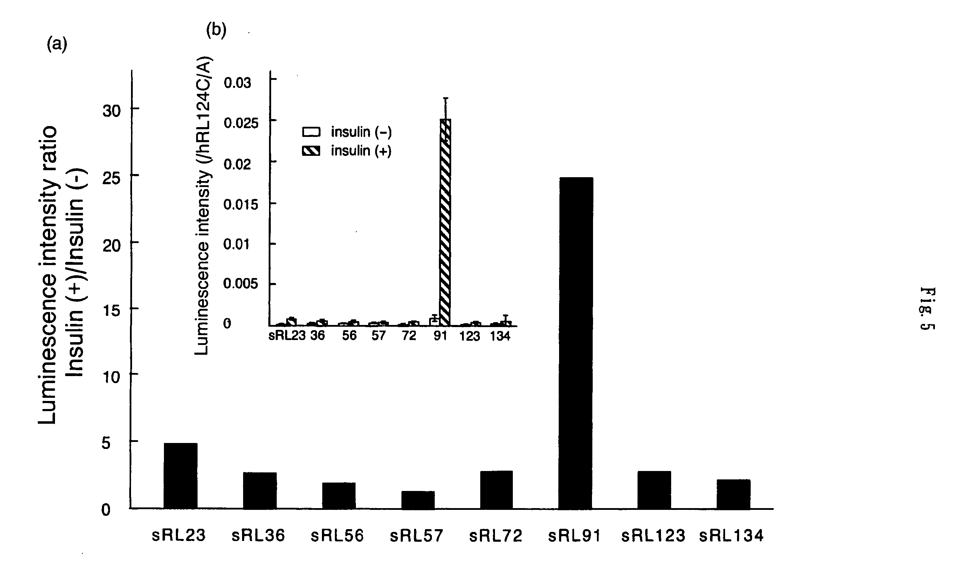

FIG. 5(a) shows the relationship between the split sites of *Renilla* luciferase and luminescence due to the protein-protein interaction (ratios of luminescence with insulin stimulation to that without insulin). The luminescence intensity of each pair of probes relative to the luminescence intensity by full-length *Renilla* luciferase (hRL124C/A) is shown in FIG. 5(b). Each measurement was conducted three times using wells different in culture plate.

The CHO-HIR cells expressing the probe (SRL91), in which *Renilla* luciferase was split between Ser91 and Tyr92, exhibited a luminescence activity of 25-fold higher with 10⁻⁷ M insulin than with its absence.

On the other hand, the probes having other split *Renilla* luciferase exhibited only a 2- 4 fold increase with 10⁻⁷ M insulin.

### Example 2

To confirm that the complementation of *Renilla* luciferase in the CHO-HIR cells expressing sRL91, was triggered by the protein-protein interaction due to protein phosphorylation, a sRL91 mutant (sRL91F) was prepared by replacing the phosphorylated site of the 941-tyrosin residue in the Y941 peptide in sRL91 with a phenylalanine residue. The N-terminal and C-terminal probes were indicated by SEQ ID NO: 6 and NO: 7, respectively.

In a similar procedure as Example 1, the luminescence intensities of the resulting sRL91F were assessed with and without insulin stimulation. Similarly, N-terminal and C-terminal sRL91 were respectively expressed from CHO-HIR cells by using the plasmids in FIG. 4(c) and FIG. 4(d), and their luminescence intensities were assessed.

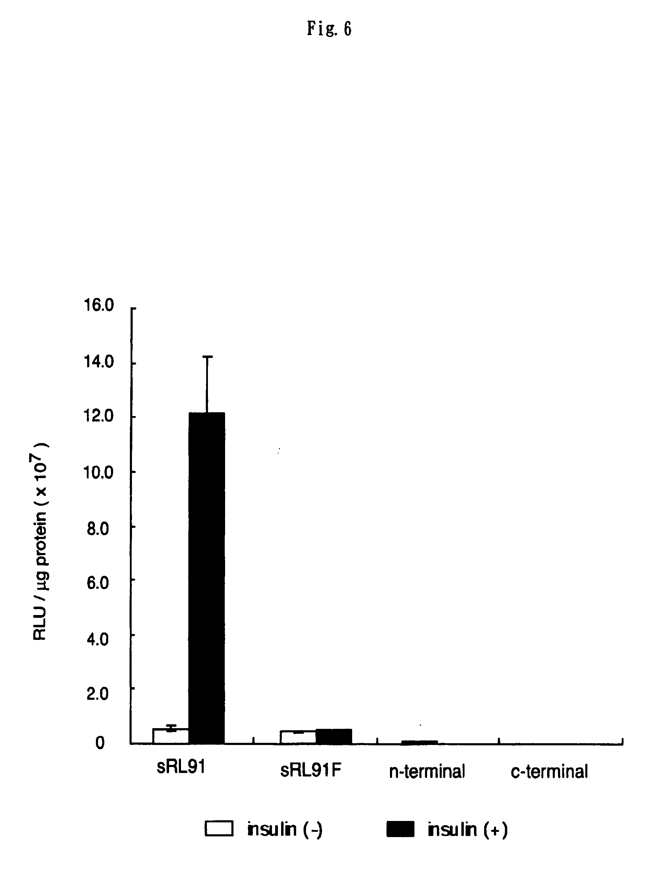

The results are shown in FIG. 6. The CHO-HIR cells expressing sRL91F did not increase its enzymatic activity upon insulin stimulation. The luminescence from the CHO-HIR cells expressing independently N-terminal and C-terminal sRL91 completely lost not only in the absence but also presence of insulin.

It has thus been confirmed that complementation of *Renilla* luciferase is induced from the protein-protein interaction between Y941 and SH2n. Accordingly, the sRL91 containing split *Renilla* luciferases obtained by splitting *Renilla* luciferase between Ser91 and Tyr92 is effective as a probe for analyzing protein-protein interaction.

### Example 3

### Time course measurement of protein-protein interaction

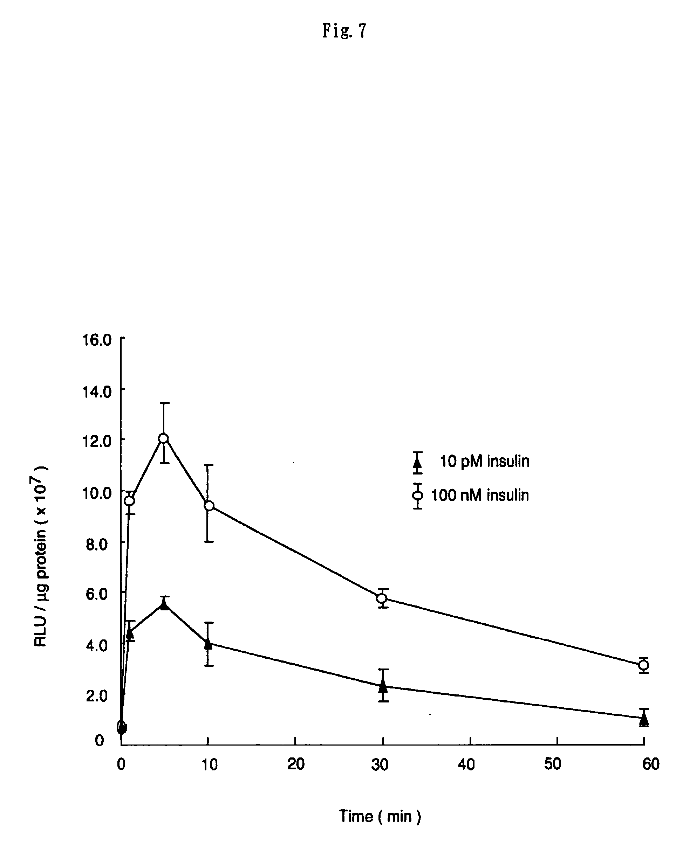

The cells expressed with sRL91 were incubated in medium supplemented with 100 nm or 10 pM insulin for 1 minute, 5 minutes, 10 minutes, 30 minutes and 60 minutes at 37°C. The luminescence of these cells was immediately assessed. The time course of interaction between Y941 and SH2n was shown in FIG. 7. The luminescence intensity increased within 5 minutes after insulin stimulation and gradually decreased afterward.

Further, the cell expressed with sRL91 was incubated in medium supplemented with 10⁻⁷ M insulin for 1 minute, 5 minutes, 10 minutes, 30 minutes and 60 minutes at 37°C. The whole cell lysate were immunoprecipitated with anti-myc antibody. The immunoblot analysis was made using anti-phosphorylated tyrosine antibody and anti-myc antibody. Antibody staining was visualized with CDP-STAR and analyzed with "LAS-1000 Image Analyzer" (Fujifilm Co., Tokyo, Japan).

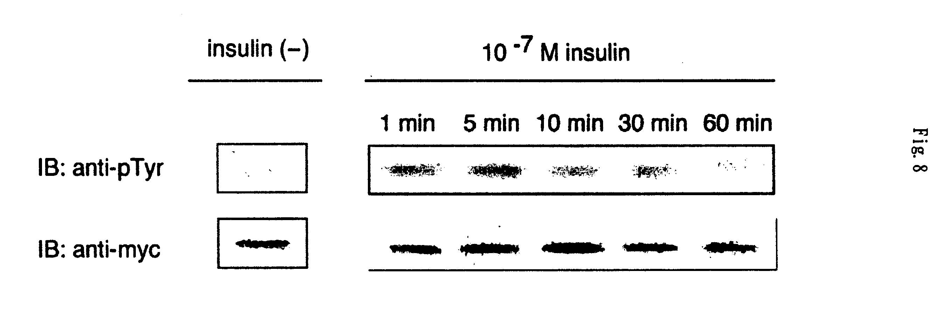

The results are shown in FIG. 8. The time course of luminescence intensity is good agreement with the results of immunoblot analysis, which suggests that the time dependence of the protein-protein interaction is due to tyrosine phosphorylation and dephosphorylation.

It has been confirmed that the luminescence activity of sRL91 directly reflects the ongoing protein-protein interaction in living cells.

### Example 4

Spontaneous emission of luminescence by protein-protein interaction-induced complementation of the split *Renilla* luciferase together with its membrane permeable substrate, coelenterazine. Therefore, noninvasive imaging of the sites and time of its occurrence of a particular protein-protein interaction in living cells was investigated by using the pair of probes of the present invention.

The protein-protein interaction between Y941 and SH2n in sRL91 expressed CHO-HIR cells was imaged. The cells were imaged at room temperature on a Carl Zeiss Axioverts 100 microscope with a cooled charged camera Micromax (Roper Scientific Inc, Tucson, AZ) controlled by Till Vision V3.02 (PHOTONICS, Planegg, Germany) with a 40 x oil immersion objective.

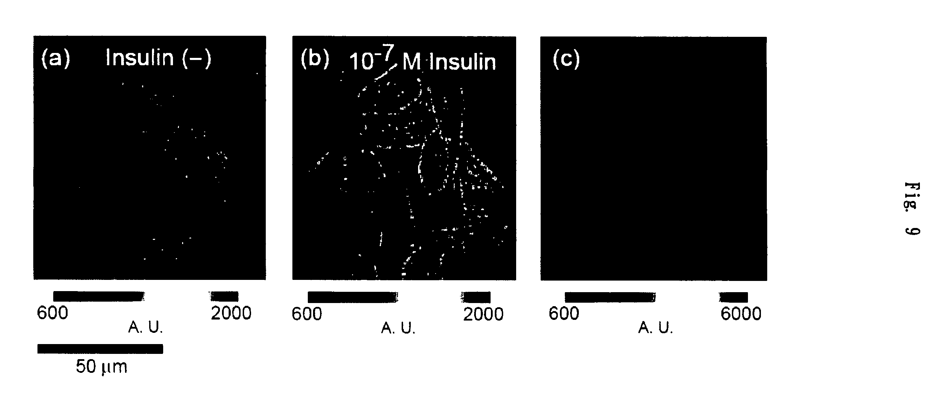

The CHO-HIR cells expressing sRL91 were thereby imaged with (b) and without (a) 10⁻⁷ M insulin stimulation as shown in FIG. 9. FIG. 9 also show microscopic image of the CHO-HIR cells expressing full-length *Renilla* luciferase (hRL124C/A).

The luminescence microscopic images were taken with a CCD camera for 300 seconds (a, b) and 60 seconds (c) as exposure times, respectively in PBS supplemented with a 20% coelenterazine substrate buffer. The luminescence intensity is represented with a color scale.

Upon insulin stimulation, luminescence emitted by complemented *Renilla* luciferase increased only beneath the cell membrane, whereas such bright contrast was not observed in the absence of insulin (FIG. 9).

This indicates that with insulin stimulation, the interaction between Y941 and SH2n occurred beneath the plasma membrane. CHO-HIR cells expressed with full-length *Renilla* luciferase (hRL124C/A) emitted luminescence uniformly throughout the cells (FIF. 9c), which precludes the possibility that the result in FIF. 9b is due to an excessive accumulation of coelenterazine in and right below the plasma membrane.

### Example 5

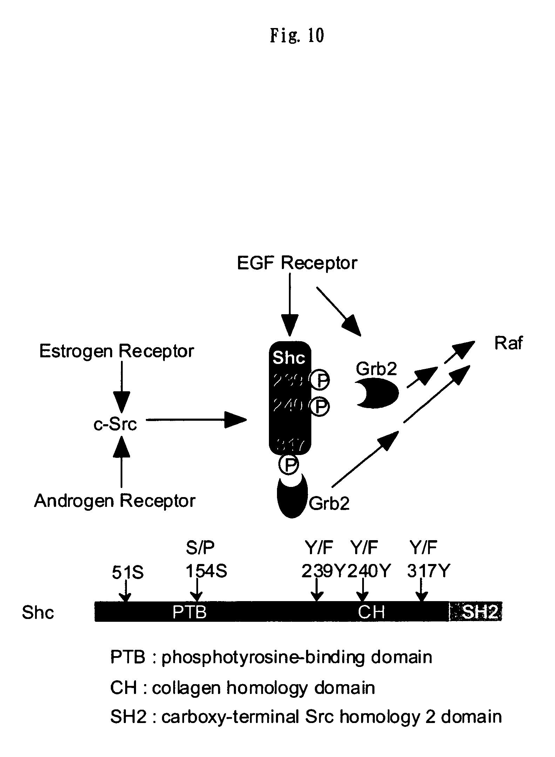

The quantitative detection of the interaction between phosphorylated adaptor protein Shc and Grb2 (FIG. 10) was succeeded by using split *Renilla* luciferase.

As a result, there are much specificity at the interaction sites between the Shc phosphorylated sites and Grb2 when stimulated by epidermal growth factor (EGF), 17β-estradiol (E2), dihydrotestosterone (DHT) or diethylstilbestrol (DES) from the outside of the cells.

The results suggest that when EGF, stimulation to a receptor type kinase, is added, the 317-tyrosine residue is phosphorylated and interacted with Grb2. This interaction requires serine in the PTB domain, presumably because the Shc is interacted with the receptor type kinase in the presence of serine in the PTB domain.

On the other hand, it has been revealed that when E2 is added, existence of serine in the PTB domain is necessary for the phosphorylation of the 239- and 240-tyrosine residues and interaction between the thus phosphorylated tyrosine residues and Grb2. The interaction between the phosphorylated 317-tyrosine residue and Grb2 does not occur in the presence of serine.

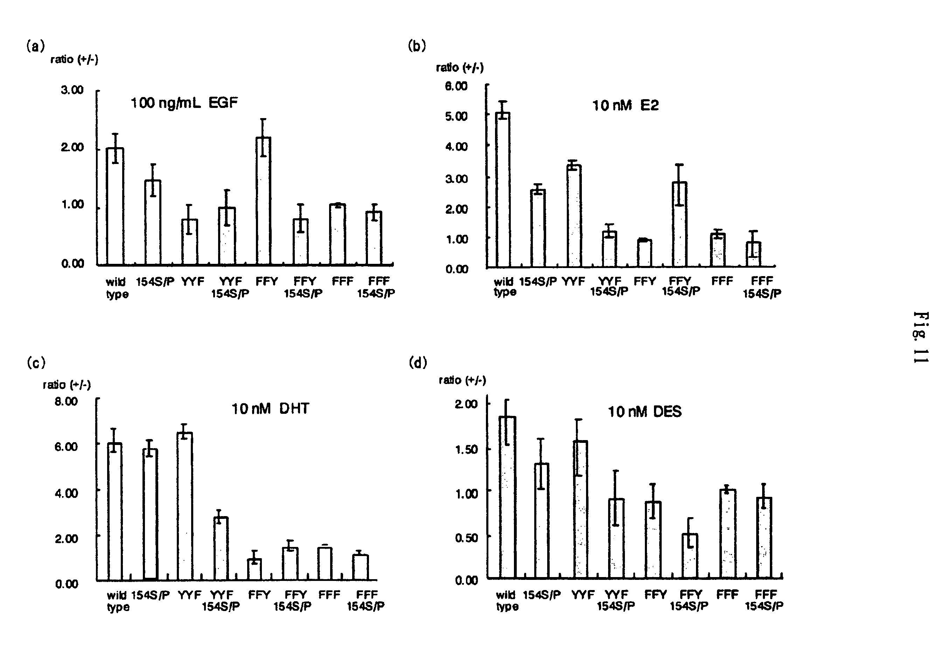

In addition, similar results were obtained (FIG. 11) when DHT was added or when DES, an estrogen receptor agonist, was added.

### Industrial Applicability

As described in full detail, the present invention provides a pair of probes for analyzing protein-protein interactions by which a protein-protein interaction in living cells can be visualized directly. The pair of probes relies on the complementation of split *Renilla* luciferase triggered by a protein-protein interaction. Unlike diffusive products involved in other complement enzyme systems (Blakely, B.T., et al., Nat. Biotechnol., 18, 218-22(2000); Rossi, F., et al., Proc. Natl. Acad. Sci. USA, 94, 8405-8410(1997); Remy, I. & Michnick, S.W., Proc. Natl. Acad. Sci. USA, 96, 5394-5399(1999); Galarneau, A., et al., Nat. Biotechnol. 20, 619-622(2002); Wehrman, T. et al., Proc. Natl. Acad. Sci. USA, 99, 3469-3474(2002)), the present bioluminescence readout by the protein-protein interaction-induced complement *Renilla* luciferase promises locating protein-protein interactions at the sites and time of its occurrence, thereby allowing analysis of protein-protein interactions *in vivo* in living cells and organisms.

## Claims

1. A pair of probes for analyzing protein-protein interactions, which comprises:
a probe A containing at least an N-terminal half polypeptide of split *Renilla* luciferase; and
a probe B containing at least the remaining C-terminal half polypeptide of split *Renilla* luciferase.

2. The pair of probes for analyzing protein-protein interactions of claim 1, wherein the probe A contains an N-terminal half polypeptide of an intein and N-split *Renilla* luciferase, and the probe B contains a C-terminal half polypeptide of the intein and C-split *Renilla* luciferase.

3. The pair of probes for analyzing protein-protein interactions of claim 1 or 2, wherein a linker sequence is linked to each of the N-terminal half polypeptide of split *Renilla* luciferase and the remaining C-terminal half polypeptide of split *Renilla* luciferase.

4. The pair of probes for analyzing protein-protein interactions of claim 3, wherein the linker sequence consists of 3 to 20 amino acid residues.

5. The pair of probes for analyzing protein-protein interactions of any one of claims 1 to 4, wherein the N-terminal half polypeptide of split *Renilla* luciferase and the remaining C-terminal half polypeptide of split *Renilla* luciferase are obtained by splitting *Renilla* luciferase between Ser91 and Tyr92.

6. A method for analyzing protein-protein interactions, which comprises
fusing a protein "a" to the probe A of any one of claims 1 to 5, and fusing a protein "b" to the probe B of any one of claims 1 to 5;
making the protein "a" fused to the probe A and the protein "b" fused to the probe B coexist in the presence of coelenterazine and oxygen; and
measuring luminescence thus emitted.

7. The method for analyzing protein-protein interactions according to claim 6, which comprises introducing a polynucleotide expressing the protein "a" fused to the probe A and a polynucleotide expressing the protein "b" fused to the probe B into cells, thereby making the protein "a" fused to the probe A and the protein "b" fused to the probe B coexist in the presence of coelenterazine and oxygen.

8. The method for analyzing protein-protein interactions according to claim 6, which comprises introducing a polynucleotide expressing the protein "a" fused to the probe A and a polynucleotide expressing the protein "b" fused to the probe B into a non-human totipotent cell, and causing ontogenesis of the cell to non-human animal, thereby making the protein "a" fused to the probe A and the protein "b" fused to the probe B coexist in the presence of coelenterazine and oxygen in any one of the cells of the animal or offspring animal thereof.

9. A non-human animal or offspring animal thereof, which is obtained by
introducing a polynucleotide expressing the protein "a" fused to the probe A and a polynucleotide expressing the protein "b" fused to the probe B into a non-human totipotent cell; and
causing ontogenesis of the cell to non-human animal.

10. A method for screening a substance, which comprises:
introducing a test sample into the non-human animal or offspring animal thereof of claim 9; and
analyzing a protein-protein interaction in the cell of the non-human animal or offspring animal thereof.


SEQUENCE LISTING

<110> Japan Science and Technology Corporation

<120> Probe for visualizing protein interaction and method of analyzing
protein-protein interaction using the same

<130> 04F025PCT

<160> 6

<210> 1
<211> 300
<212> PRT
<213> Renilla reniformis
<400> 1
Met Thr Ser Lys Val Tyr Asp Pro Glu Gln Arg Lys Arg Met Ile
1               5                   10                  15
Thr Gly Pro Gln Trp Trp Ala Arg Cys Lys Gln Met Asn Val Leu
16 20 25 30
Asp Ser Phe Ile Asn Tyr Tyr Asp Ser Glu Lys His Ala Glu Asn
31 35 40 45
Ala Val Ile Phe Leu His Gly Asn Ala Ala Ser Ser Tyr Leu Trp
46 50 55 60
Arg His Val Val Pro His Ile Glu Pro Val Ala Arg Cys Ile Ile
61 65 70 75
Pro Asp Leu Ile Gly Met Gly Lys Ser Gly Lys Ser Gly Asn Gly
76 80 85 90
Ser Tyr Arg Leu Leu Asp His Tyr Lys Tyr Leu Thr Ala Trp Phe
91 95 100 105
Glu Leu Leu Asn Leu Pro Lys Lys Ile Ile Phe Val Gly His Asp
106 110 115 120
Trp Gly Ala Cys Leu Ala Phe His Tyr Cys Tyr Glu His Gln Asp
121 125 130 135
Lys Ile Lys Ala Ile Val His Ala Glu Ser Val Val Asp Val Ile
136 140 145 150
Glu Ser Trp Asp Glu Trp Pro Asp Ile Glu Glu Asp Ile Ala Leu
151 155 160 165
Ile Lys Ser Glu Glu Gly Glu Lys Met Val Leu Glu Asn Asn Phe
166 170 175 180
Phe Val Glu Thr Met Leu Pro Ser Lys Ile Met Arg Lys Leu Glu
181 185 190 195
Pro Glu Glu Phe Ala Ala Tyr Leu Glu Pro Phe Lys Glu Lys Gly
196 200 205 210
Glu Val Arg Arg Pro Thr Leu Ser Trp Pro Arg Glu Ile Pro Leu
211 215 220 225
Val Lys Gly Gly Lys Pro Asp Val Val Gln Ile Val Arg Asn Tyr

226                 230                      235                      240
Asn Ala Tyr Leu Arg Ala Ser Asp Asp Leu Pro Lys Met Phe Ile
241                 245                      250                      255
Glu Ser Asp Pro Gly Phe Phe Ser Asn Ala Ile Val Glu Gly Ala
256                 260                      265                      270
Lys Lys Phe Pro Asn Thr Glu Phe Val Lys Val Lys Gly Leu His
271                 275                      280                      285
Phe Ser Gln Glu Asp Ala Pro Asp Glu Met Gly Asn Tyr Ile Gln
286                 290                      295                      300

<210> 2
<211> 13
<212> PRT
<213> Artificial Sequence
<223> Synthesized Oligopeptide
<400> 2
Thr Glu Glu Ala Tyr Met Lys Met Asp Leu Gly Pro Gly
1               5                   10

<210> 3
<211> 300
<212> PRT
<213> Renilla reniformis
<400> 3
Met Thr Ser Lys Val Tyr Asp Pro Glu Gln Arg Lys Arg Met Ile
1               5                   10                  15
Thr Gly Pro Gln Trp Trp Ala Arg Cys Lys Gln Met Asn Val Leu
16              20                  25                  30
Asp Ser Phe Ile Asn Tyr Tyr Asp Ser Glu Lys His Ala Glu Asn
31              35                  40                  45
Ala Val Ile Phe Leu His Gly Asn Ala Ala Ser Ser Tyr Leu Trp
46              50                  55                  60
Arg His Val Val Pro His Ile Glu Pro Val Ala Arg Cys Ile Ile
61              65                  70                  75
Pro Asp Leu Ile Gly Met Gly Lys Ser Gly Lys Ser Gly Asn Gly
76              80                  85                  90
Ser Tyr Arg Leu Leu Asp His Tyr Lys Tyr Leu Thr Ala Trp Phe
91              95                  100                 105
Glu Leu Leu Asn Leu Pro Lys Lys Ile Ile Phe Val Gly His Asp
106             110                 115                 120
Trp Gly Ala Ala Leu Ala Phe His Tyr Cys Tyr Glu His Gln Asp
121             125                 130                 135
Lys Ile Lys Ala Ile Val His Ala Glu Ser Val Val Asp Val Ile
136             140                 145                 150
Glu Ser Trp Asp Glu Trp Pro Asp Ile Glu Glu Asp Ile Ala Leu
151             155                 160                 165

Ile Lys Ser Glu Glu Gly Glu Lys Met Val Leu Glu Asn Asn Phe
166 170 175 180
Phe Val Glu Thr Met Leu Pro Ser Lys Ile Met Arg Lys Leu Glu
181 185 190 195
Pro Glu Glu Phe Ala Ala Tyr Leu Glu Pro Phe Lys Glu Lys Gly
196 200 205 210
Glu Val Arg Arg Pro Thr Leu Ser Trp Pro Arg Glu Ile Pro Leu
211 215 220 225
Val Lys Gly Gly Lys Pro Asp Val Val Gln Ile Val Arg Asn Tyr
226 230 235 240
Asn Ala Tyr Leu Arg Ala Ser Asp Asp Leu Pro Lys Met Phe Ile
241 245 250 255
Glu Ser Asp Pro Gly Phe Phe Ser Asn Ala Ile Val Glu Gly Ala
256 260 265 270
Lys Lys Phe Pro Asn Thr Glu Phe Val Lys Val Lys Gly Leu His
271 275 280 285
Phe Ser Gln Glu Asp Ala Pro Asp Glu Met Gly Asn Tyr Ile Gln
286 290 295 300

<210> 4
<211> 18
<212> PRT
<213> Artificial Sequence
<223> Synthesized Oligopeptide
<400> 4
Cys Leu Ser Leu Ala Ser Asn Asn Gly Asn Gly Arg Asn Gly Ala
1               5                   10                  15
Ser Leu Glu
16

<210> 5
<211> 17
<212> PRT
<213> Artificial Sequence
<223> Synthesized Oligopeptide
<400> 5
Pro Arg Gly Asn Asn Gly Gly Asn Asn Asp Val Met Ala Ile Ala
1               5                   10                  15
Ala Asn
16

<210> 6
<211> 133
<212> PRT
<213> Artificial Sequence

<223> Synthesized Oligopeptide
<400> 6
Met Thr Ser Lys Val Tyr Asp Pro Glu Gln Arg Lys Arg Met Ile
1               5                   10                  15
Thr Gly Pro Gln Trp Trp Ala Arg Cys Lys Gln Met Asn Val Leu
16              20                  25                  30
Asp Ser Phe Ile Asn Tyr Tyr Asp Ser Glu Lys His Ala Glu Asn
31              35                  40                  45
Ala Val Ile Phe Leu His Gly Asn Ala Ala Ser Ser Tyr Leu Trp
46              50                  55                  60
Arg His Val Val Pro His Ile Glu Pro Val Ala Arg Cys Ile Ile
61              65                  70                  75
Pro Asp Leu Ile Gly Met Gly Lys Ser Gly Lys Ser Gly Asn Gly
76              80                  85                  90
Ser Cys Leu Ser Leu Ala Ser Asn Asn Gly Asn Gly Arg Asn Gly
91              95                  100                 105
Ala Ser Leu Glu Thr Glu Glu Tyr Met Lys Met Asp Leu Gly Pro
106             110                 115                 120
Gly Thr Arg Glu Gln Lys Leu Ile Ser Glu Glu Asp Leu
121             125                 130

<210> 7
<211> 352
<212> PRT
<213> Artificial Sequence
<223> Synthesized Oligopeptide
<400> 7
Met Asp Ala Glu Trp Tyr Trp Gly Asp Ile Ser Arg Glu Glu Val
1               5                   10                  15
Asn Glu Lys Leu Arg Asp Thr Ala Asp Gly Thr Phe Leu Val Arg
16              20                  25                  30
Asp Ala Ser Thr Lys Met His Gly Asp Tyr Thr Leu Thr Leu Arg
31              35                  40                  45
Lys Gly Gly Asn Asn Lys Leu Ile Lys Ile Phe His Arg Asp Gly
46              50                  55                  60
Lys Tyr Gly Phe Ser Asp Pro Leu Thr Phe Asn Ser Val Val Glu
61              65                  70                  75
Leu Ile Asn His Tyr Arg Asn Glu Ser Leu Ala Gln Tyr Asn Pro
76              80                  85                  90
Lys Leu Asp Val Lys Leu Leu Tyr Pro Val Ser Lys Tyr Gln Gln
91              95                  100                 105
Pro Arg Gly Asn Asn Gly Gly Asn Asn Asp Val Met Ala Ile Ala
106             110                 115                 120
Ala Asn Tyr Arg Leu Leu Asp His Tyr Lys Tyr Leu Thr Ala Trp
121             125                 130                 135
Phe Glu Leu Leu Asn Leu Pro Lys Lys Ile Ile Phe Val Gly His
136             140                 145                 150

Asp Trp Gly Ala Cys Leu Ala Phe His Tyr Ser Tyr Glu His Gln
151 155 160 165
Asp Lys Ile Lys Ala Ile Val His Ala Glu Ser Val Val Asp Val
166 170 175 180
Ile Glu Ser Trp Asp Glu Trp Pro Asp Ile Glu Glu Asp Ile Ala
181 185 190 195

Leu Ile Lys Ser Glu Glu Gly Glu Lys Met Val Leu Glu Asn Asn
196 200 205 210
Phe Phe Val Glu Thr Met Leu Pro Ser Lys Ile Met Arg Lys Leu
211 215 220 225
Glu Pro Glu Glu Phe Ala Ala Tyr Leu Glu Pro Phe Lys Glu Lys
226 230 235 240
Gly Glu Val Arg Arg Pro Thr Leu Ser Trp Pro Arg Glu Ile Pro
241 245 250 255
Leu Val Lys Gly Gly Lys Pro Asp Val Val Gln Ile Val Arg Asn
256 260 265 270
Tyr Asn Ala Tyr Leu Arg Ala Ser Asp Asp Leu Pro Lys Met Phe
271 275 280 285
Ile Glu Ser Asp Pro Gly Phe Phe Ser Asn Ala Ile Val Glu Gly
286 290 295 300
Ala Lys Lys Phe Pro Asn Thr Glu Phe Val Lys Val Lys Gly Leu
301 305 310 315
His Phe Ser Gln Glu Asp Ala Pro Asp Glu Met Gly Lys Tyr Ile
316 320 325 330
Lys Ser Phe Val Glu Arg Val Leu Lys Asn Glu Gln Pro Arg Asp
331 335 340 345
Tyr Lys Asp Asp Val Val Lys
346 350